**Europäisches Patentamt**

**European Patent Office**

**Office européen des brevets**

(19)

(11) Veröffentlichungsnummer: **0 083 767**
**B1**

(12) **EUROPÄISCHE PATENTSCHRIFT**

(45) Veröffentlichungstag der Patentschrift:
**20.03.85**

(51) Int. Cl.⁴: **C 09 K 15/08, A 61 K 7/42**

(21) Anmeldenummer: **82111709.0**

(22) Anmeldetag: **16.12.82**

(54) **Ethoxylierte Alpha-Naphthole als Lichtschutzmittel.**

(30) Priorität: **07.01.82 DE 3200199**

(43) Veröffentlichungstag der Anmeldung:
**20.07.83 Patentblatt 83/29**

(45) Bekanntmachung des Hinweises auf die Patenterteilung:
**20.03.85 Patentblatt 85/12**

(84) Benannte Vertragsstaaten:
**AT BE CH DE FR GB IT LI NL**

(56) Entgegenhaltungen:
**Keine**

(73) Patentinhaber: **BASF Aktiengesellschaft,
Carl-Bosch-Strasse 38, D-6700 Ludwigshafen (DE)**

(72) Erfinder: **Oppenlaender, Knut, Dr., Otto-Dill-Strasse 23,
D-6700 Ludwigshafen (DE)**
Erfinder: **Strickler, Rainer, Dr., Schroederstrasse 14,
D-6900 Heidelberg (DE)**
Erfinder: **Selb, Karl, Dr., Kriemholdstrasse 36,
D-6940 Weinheim (DE)**
Erfinder: **Naegele, Paul, Wiesenstrasse 9,
D-6708 Neuhofen (DE)**

## Beschreibung

Die Erfindung betrifft die Verwendung von ethoxylierten α-Naphtholen als Lichtschutzmittel sowohl in kosmetischen Präparationen als auch in sonstigen technischen Bereichen.

Es ist bekannt, dass der als UV-B-Strahlung bezeichnete Bereich zwischen 280 und 320 nm des Sonnenlichts oder künstlicher Lichtquellen für die Erythembildung der menschlichen Haut verantwortlich ist. Das Maximum der Wirksamkeit der UV-Strahlung für die Erythembildung liegt bei 297 nm, wenn die Strahlungsintensität für alle Wellenlängen gleich gross ist. Beim Sonnenlicht mit Strahlung unterschiedlicher Intensität ist dieses Maximum auf 308 nm verschoben. Durch geeignete Filtersubstanzen für den UV-B-Bereich gelingt es, die Erythembildung zu verhindern oder zumindest zu verzögern. Die Pigmentbildung der Haut, d.h. die Bräunung, soll dagegen gewährleistet bleiben.

Die UV-Strahlung ist darüber hinaus auch eine wichtige Einflussgrösse bei der Alterung von Polymeren und kann beispielsweise die Umwandlung bestimmter Farbstoffe bewirken, so dass auch für solche Produkte Filtersubstanzen als Stabilisatoren nahezu unentbehrlich sind.

In den vergangenen 40 Jahren ist eine grosse Anzahl von chemischen Verbindungen auf ihre Filterwirkung im UV-B-Bereich hin untersucht worden. Ob es sich jedoch bei einer im UV-Gebiet absorbierenden Substanz auch für die menschliche Haut um einen brauchbaren Sonnenschutzfilter handelt, wird noch von anderen Faktoren entscheidend bestimmt. Neben der hohen Filterwirksamkeit im erythemalen Bereich soll die Substanz eine verhältnismässig hohe Durchlässigkeit im UV-A-Bereich aufweisen. Sie sollte auch eine möglichst ideale Haut- und Schleimhautverträglichkeit besitzen und darf nicht toxisch sein. Schliesslich soll sie auch nicht oxidationsempfindlich sein und durch UV-Bestrahlung keine Veränderungen oder Verfärbungen erleiden. Eine entsprechende Zubereitung soll lagerstabil sein, keinen Eigengeruch aufweisen und mit den üblicherweise verwendeten kosmetischen Rohstoffen verträglich sein.

Die bekannten UV-Filtersubstanzen weisen häufig insofern Nachteile auf, als sie beim Lagern gegen UV-Strahlung oder sichtbare Strahlung und/oder Luft instabil sind, in gefärbte Zersetzungsprodukte übergehen, die Wäsche anschmutzen oder sogar hautschädlich sein können. In der Praxis haben sich nur verhältnismässig wenige Substanzen, die den angegebenen Ansprüchen mehr oder weniger gut entsprechen, durchgesetzt, wie beispielsweise der „Chemischen Rundschau", 24, 1097 ff. (1971), entnommen werden kann.

Weiterhin ist beispielsweise aus der DE-PS Nr. 1543387 bekannt, dass polyalkoxylierte p-Aminobenzoesäureester als Lichtschutzmittel verwendet werden können. Die Polyalkoxylierung erfolgt dabei durch Umsetzung der entsprechenden Carbonsäuren oder Carbonsäureester mit Alkylenoxiden in Gegenwart von Alkalien. Die Anlagerung der Alkylenoxide kann sowohl an der Carbonsäure- als auch an der Aminogruppe erfolgen. Ein hervorzuhebender Vorteil der Verbindungen liegt darin, dass sie relativ gut wasserlöslich sind, insbesondere bei Ethoxylierungsgraden von $\geq$ 6.

Das Ziel der Erfindung wurde überraschenderweise mit der Verwendung von ethoxylierten α-Naphtholen erreicht, wie gemäss den Patentansprüchen definiert ist.

Es handelt sich um α-Naphthol, das 1- bis 25fach, vorzugsweise 4- bis 15fach ethoxyliert ist.

Die erfindungsgemäss zu verwendenden Ethoxylate sind aus leicht zugänglichen Rohstoffen in einfacher Weise herstellbar.

Man setzt in erster Stufe α-Naphthol mit Ethylenoxid in Anwesenheit eines Säurekatalysators, bevorzugt einer Lewis-Säure, wie Zinkchlorid, Aluminiumchlorid oder Eisen(III)chlorid bei Temperaturen zwischen 70 und 160°C und Drucken zwischen 1 und 10 bar zu O-Hydroxyethyl-α-naphthol um.

Dieses einfach ethoxylierte α-Naphthol ist bereits im erfindungsgemässe Sinne wirksam.

Zweckmässig ist es aber, diese Verbindungen weiter umzusetzen.

Das O-Hydroxyethyl-α-naphthol kann in üblicher Weise mit Ethylenoxid zu höher ethoxylierten Produkten umgesetzt werden. Als Katalysator für die Ethoxylierung verwendet man in bekannter Weise Hydroxide, Alkoholate oder Oxide der Elemente der 1. und 2. Hauptgruppe des Periodensystems, insbesondere des Natriums oder Kaliums. Die Umsetzung wird bevorzugt bei Temperaturen zwischen 80 und 160°C in Anwesenheit vorzugsweise von 0,1 bis 5 Gew.-% der alkalisch reagierenden Substanzen und bei Drücken zwischen 1 und 10 bar durchgeführt. Die Reaktionszeiten betragen im allgemeinen 1 bis 20, vorzugsweise 4 bis 8 h. Der Verlauf der Ethoxylierung kann mit üblichen analytischen Methoden überwacht werden. Diese Methoden sind altbekannt und bedürfen daher an dieser Stelle keiner speziellen Erläuterung.

Das Ende der Reaktion kann daran erkannt werden, dass bei konstanter Temperatur keine weitere Druckabnahme und somit keine weitere Ethylenoxidaufnahme mehr stattfindet.

Man kann aber auch α-Naphthol sofort mit der gesamten benötigten Menge an Ethylenoxid in alkalischem Medium umsetzen. Auch diese Methode ist altbekannt. Bevorzugt arbeitet man hier bei Temperaturen von 80 bis 140°C.

Die alkalisch reagierenden Mittel können am Ende des Reaktionsprozesses nach dem Abkühlen des Gemisches auf 50 bis 20°C durch Zusatz verdünnter Carbonsäuren unter Rühren neutralisiert werden.

Ethoxylierte α-Naphthole sind als Substanzen aus der DE-PS Nr. 1191103 bekannt. Sie werden dort als Polymerisationsbeschleuniger beschrieben. Hinsichtlich einer möglichen Verwendung als Lichtschutzmittel ist dagegen aus der Literatur nichts bekannt.

Die erfindungsgemäss zu verwendenden Verbindungen absorbieren mit sehr guter Extinktion den UV-B-Bereich. Durch Variation des Ethoxylierungsgrades können Verbindungen von bestimmter Konsistenz und mit gewünschten Eigenschaften im Hinblick auf die Löslichkeit hergestellt werden.

Die Palette der bisher verfügbaren Lichtschutzmittel wird durch die erfindungsgemäss zu verwendenden und chemisch leicht zugänglichen Verbindungen in vorteilhafter Weise bereichert.

α-Naphthole mit Oxethylierungsgraden von weniger als 5 zeigen eine gute Löslichkeit in Alkoholen, natürlichen und synthetischen Estern und anderen kosmetischen Grundstoffen. So können durch den Ethoxylierungsgrad die geforderten physikalischen Eigenschaften individuell eingestellt werden. Ein besonderer Vorteil besteht darin, dass die zu verwendenden Verbindungen nicht verseifbar sind und damit eine hohe chemische Stabilität aufweisen. Bedingt durch ihren chemischen Aufbau besitzen sie zudem noch Tensideigenschaften, wodurch kosmetische Formulierungen (Emulsionen) zusätzlich stabilisert werden können.

Mit den Verbindungen können hohe Lichtschutzfaktoren erzielt werden. Die Filterwirkung für den UV-B-Bereich in wässerigen Lösungen kann allgemein zur Stabilisierung von kosmetischen Zubereitungen, Kunststoffen, Farbstoffen, Lösungs- oder Anstrichmitteln benutzt werden. Sie können auch in Kombination mit anderen Lichtschutzmitteln Verwendung finden.

Kosmetische Präparate oder Zubereitungen enthalten erfindungsgemäss bevorzugt 0,1 bis 15 Gew.-% an den Verbindungen. Technisch besonders günstige Bereiche stellen Prozentsätze von 2 bis 8 Gew.-% dar. Die kosmetischen Zubereitungen enthalten ansonsten die üblichen festen, halbfesten oder flüssigen Trägerstoffe oder Verdünnungsmittel, Gemische aus diesen und gegebenenfalls sonstige übliche kosmetische Hilfsstoffe.

Von der Art des Trägers, Hilfsstoffes oder Verdünnungsmittels hängt es ab, ob das fertige lichtschutzmittelhaltige Präparat beispielsweise eine Lösung, ein Öl, eine Creme, eine Salbe, eine Lotion, ein Gel oder ein Pulver ist. Derartige Zubereitungen können beispielsweise der Zeitschrift „Fette und Seifen", 53. Jahrgang, S. 694 bis 699 (1951), der Zeitschrift „Seifen, Öle, Fette, Wachse", 1955, S. 147, oder H. Janistyn, „Handbuch der Kosmetika und Riechstoffe", 3. Bd. (1973), entnommen werden.

Üblicherweise verwendete kosmetische Hilfsstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel oder Parfüms. Grundlage für Sonnenschutzöle sind beispielsweise pflanzliche Öle, wie Erdnussöl, Olivenöl, Sesamöl, Baumwollsamenöl, Kokosöl, Traubenkernöl, Ricinusöl oder Mineralöle, wie Vaselinöl, oder insbesondere flüssiges Paraffin, synthetische Fettsäureester und Glyceride.

Grundlage für Seifen sind beispielsweise Vaseline, Lanolin, Eucerin oder Polyethylenglykole.

Grundlage für Cremes sind beispielsweise fettreiche Cremes, Glycerin-, Polysaccharid-, Tylosecreme, für Cremes auf Basis von Fetten und Wachsen Ethylalkohol, Lanolincreme, Kakaobutter, Bienenwachs, Stearinsäure, Stearylalkohol, Glycerinmonostearat, native oder mineralische Öle und Fette.

Grundlage für Emulsionen sind beispielsweise Mischungen aus Stearylglykol, einem pflanzlichen und/oder Mineralöl, wie Mandelöl, Paraffinöl und Vaseline, und Wasser oder Mischungen aus Ethylalkohol, Wasser, Lanolin und Tragant, oder Mischungen aus Ethylalkohol, Stearin, Wasser oder Tragant, Glycerin, Alkohol und Wasser oder Mischungen aus Stearinsäure, Paraffinöl, Propyl- oder Isopropylalkohol und Wasser.

Bei der Stabilisierung von Kunststoffen, Farbstoffen und anderen lichtempfindlichen technischen Produkten, setzt man die erfindungsgemäss zu verwendenden Produkte im allgemeinen zu 0,1 bis 5 Gew.-% in die entsprechenden Stoffe durch Mischen ein.

Die nun folgenden Beispiele erläutern die Erfindung, ohne sie zu beschränken.

*Beispiele*

*Polyethylenglykol-1-naphthylether   (allgemeine Vorschrift)*

144 g 1-Naphthol (1 mol) und 0,15 g KOH-Pulver werden im Rührautoklaven vorgelegt, fünfmal mit Stickstoff gespült und auf 120 bis 125°C aufgeheizt. Dann werden x g Ethylenoxid (y mol) portionsweise bis zu einem Druck von 8 bar aufgepresst. Nach der Aufpresszeit lässt man 3 h nachrühren. Eventuell kann das so erhaltene Produkt durch Behandeln mit Bleicherde (5 Gew.-%) bei 80 bis 90°C über ½ h aufgehellt werden.

| Beispiel Nr. | 1-Naphthol +y Ethylenoxid y | x g Ethylenoxid pro mol 1-Naphthol x | Auswaage (g) (Theorie) | OHZ (Theorie) | UV-Spektrum | |
|---|---|---|---|---|---|---|
| | | | | | λ (nm) | $\left(\dfrac{1000\ cm^2}{mol}\right)$ |
| 1 | 5,3 | 233,2 | 352 (377,2) | 148 (148) | 293,6 | 5650 |
| 2 | 5,5 | 242,0 | 381 (386,0) | 157 (145) | 293,6 | 5667 |
| 3 | 6,0 | 264,0 | 394 (408,0) | 149 (137) | 293,5 | 5705 |
| 4 | 6,5 | 286,0 | 405 (430,0) | 140 (131) | 293,4 | 5740 |
| 5 | 7,0 | 308,0 | 447 (452,0) | 133 (125) | 293,5 | 5755 |
| 6 | 8,3 | 365,2 | 478 (509,2) | 121 (110) | 293,4 | 5840 |
| 7 | 10,6 | 466,4 | 581 (610,4) | 107 (92) | 293,4 | 5980 |

*Rezeptbeispiele*

| *Lichtschutzgel* | *Teile* |
|---|---|
| α-Naphthol + 8,3 Ethylenoxid (EO) gemäss Beispiel 6 | 10,0 |
| hydriertes Ricinusöl + 50 EO | 1,0 |
| Ethanol | 15,0 |
| Polyacrylsäure | 0,8 |
| Triethanolamin | 1,0 |
| Parfümöl | 0,2 |
| Wasser | 72,0 |

| *Lichtschutzcreme* | *Teile* |
|---|---|
| α-Naphthol + 5,5 EO gemäss Beispiel 2 | 5,0 |
| Glycerinmonostearat | 7,0 |
| Fettalkohol + 25 EO | 2,0 |
| Fettalkohol + 6 EO | 2,0 |
| Parfümöl | 4,0 |
| Fettsäuretriglycerid | 4,0 |
| 1,2-Propylenglykol | 3,0 |
| Konservierungsmittel | 0,5 |
| Paraffinöl | 0,2 |
| Wasser | 70,3 |

| *Lichtschutzemulsion* | *Teile* |
|---|---|
| α-Naphthol + 6,5 EO gemäss Beispiel 4 | 10,0 |
| Glycerinmonostearat + Natriumstearat | 6,0 |
| Fettalkohol + 25 EO | 2,0 |
| Fettalkohol + 6 EO | 2,0 |
| Paraffinöl | 5,0 |
| Fettsäuretriglycerid | 5,0 |
| 1,2-Propylenglykol | 3,0 |
| Konservierungsmittel | 0,5 |
| Parfümöl | 0,2 |
| Wasser | 66,3 |

**Patentansprüche**

1. Verwendung von 1- bis 25fach ethoxyliertem α-Naphthol als Lichtschutzmittel.

2. Verwendung von ethoxyliertem α-Naphthol nach Anspruch 1 als Lichtschutzmittel in kosmetischen Präparationen.

3. Verwendung von ethoxyliertem α-Naphthol nach Anspruch 1 als technische Lichtschutzmittel oder UV-Stabilisatoren.

4. Verwendung von ethoxyliertem α-Naphthol nach den Ansprüchen 1 und 2 in kosmetischen Präparationen in Mengen von — bezogen auf die Präparationen — 0,1 bis 15 Gew.-%.

**Revendications**

1. Utilisation d'α-naphtols 1 à 25 fois éthoxylés comme agents de protection contre la lumière.

2. Utilisation d'un α-naphtol éthoxylé selon la revendication 1 comme agent protecteur contre la lumière dans des compositions cosmétiques.

3. Utilisation d'un α-naphtol éthoxylé selon la revendication 1 comme agent de protection contre la lumière ou de stabilisation vis-à-vis des rayons ultraviolets dans des produits industriels.

4. Utilisation d'un α-naphtol éthoxylé selon les revendications 1 et 2 dans des compositions cosmétiques en des proportions de 0,1 à 15% de leur poids.

**Claims**

1. The use of α-naphthol, oxyethylated with from 1 to 25 mol of ethylene oxide, as a UV adsorbent.

2. The use of oxyethylated α-naphthol as claimed in Claim 1 as a sunscreen agent in cosmetic preparations.

3. The use of oxyethylated α-naphthol as claimed in Claim 1 as an industrial light stabilizer or UV stabilizer.

4. The use of oxyethylated α-naphthol as claimed in Claims 1 and 2 in cosmetic preparations in amounts of from 0.1 to 15% by weight, based on the preparation.